# EUROPÄISCHE PATENTSCHRIFT

(11) **EP 3 280 371 B1**
(45) Veröffentlichungstag und Bekanntmachung des Hinweises auf die Patenterteilung: **05.06.2019**
(21) Anmeldenummer: 16719025.5
(22) Anmeldetag: 08.04.2016
(51) Int. Cl.: A61F 13/02, A61K 9/70, A61L 15/44, A61F 7/00, H05B 3/34, A61F 13/00

(54) **ELEKTRISCH ERWÄRMBARES PFLASTER**
ELECTRICALLY HEATABLE PLASTER
PANSEMENT ELECTRIQUEMENT CHAUFFABLE

(30) Priorität: 08.04.2015 EP 15162818
(43) Veröffentlichungstag der Anmeldung: 14.02.2018
(73) Patentinhaber: LTS Lohmann Therapie-Systeme AG, 56626 Andernach (DE)
(72) Erfinder: KLAFFENBACH, Peter, 41564 Kaarst (DE)
(74) Vertreter: Held, Stephan
(86) Internationale Anmeldenummer: PCT/EP2016/057756
(87) Internationale Veröffentlichungsnummer: WO 2016/162481

(56) Entgegenhaltungen:
- EP-A1- 0 212 096
- EP-A2- 0 463 516
- WO-A2-03/039417
- WO-A2-2005/044141

## Beschreibung

Die Erfindung betrifft Pflaster, einschließlich Wirkstoffpflaster, und Verfahren zu deren Herstellung. Insbesondere betrifft die Erfindung Heft- und Wirkstoffpflaster, die elektrisch erwärmbar sind.

Der Wärme wird eine heilende Wirkung zugesprochen. Daher gehören Wärmetherapien zu den ältesten medizinischen Verfahren. Anders als die therapeutische Hyperthermie wird die medizinische Wärmetherapie lokal eingesetzt, beispielsweise bei Erkrankungen des Bewegungsapparates oder bei Überlastungsschäden. Der Wärme werden in der Medizin vor allem folgende Wirkungen zugesprochen: Muskelentspannung, Verbesserung der Durchblutung, Verminderung der Viskosität der Gelenkflüssigkeit, Verbesserung der Dehnbarkeit des kollagenen Bindegewebes und Schmerzlinderung.

Für eine lokale Wärmetherapie werden üblicherweise bestimmte Träger, die zuvor erhitzt wurden, als Latentwärmespeicher für mehrere Minuten bis einige Stunden auf die zu behandelnden Körperareale aufgebracht. Beispiele für Latentwärmespeicher sind Körnerkissen, Fangopackungen oder wärmespeichernde Gele. Für eine lokale Wärmetherapie können aber auch Wärmekissen verwendet werden, deren Inhaltsstoffe über einen beschleunigten Oxidationsprozess bis zu 24 Stunden Wärme abgeben. Weiterhin können auch spezifische Hautrezeptoren mit einem Capsaicin enthaltenden Trägerstoff, beispielsweise einer Salbe oder einem Pflaster, gereizt werden, um ein subjektives Wärmegefühl zu erzeugen.

Für eine lokale Wärmetherapie sind Pflaster besonders geeignet, weil Sie mit ihrer haftklebenden Fläche auf oder an der Haut eines Patienten befestigt werden können, so dass sie selbst bei Bewegungen des Patienten

nicht verrutschen und ihren Kontakt zur Hautoberfläche des Patienten behalten.

Es ist auch bekannt, dass eine Wärmezufuhr die transdermale Verabreichung eines pharmazeutischen Wirkstoffs verbessern kann. Allerdings erlauben für diese Zwecke verwendete Latentwärmespeicher keine kontrollierte und/oder steuerbare Wärmezufuhr, denn beispielsweise ist weder eine präzise Einstellung einer Zieltemperatur noch das konstante Halten einer bestimmten Zieltemperatur über einen längeren Zeitraum von bis zu mehreren Stunden möglich. Sofern der Latentwärmespeicher fest in einem Pflaster integriert ist, ist auch eine Unterbrechung der Wärmezufuhr, ohne das Pflaster von der Haut des Patienten entfernen zu müssen, nicht möglich.

Der vorliegenden Erfindung lag daher die Aufgabe zugrunde, ein Pflaster für eine lokale Wärmezufuhr bereitzustellen, wobei das Pflaster eine steuerbare Temperaturführung auch über mehrere Stunden hinweg ermöglicht, ohne das Pflaster von seiner Applikationsstelle entfernen zu müssen.

Die Druckschrift WO 99/15101 A1 offenbart eine mehrschichtige Wundauflage mit die Wundheilung fördernden und schmerzlindernden Eigenschaften. Diese Wundauflage weist mindestens eine elektrisch gut leitende Schicht auf, die flexibel ist und aus versilberten Fasern oder einer Mischung aus versilberten Fasern und nicht metallisierten Fasern zusammengesetzt ist.

Aus der Offenlegungsschrift WO 03/039417 A2 ist eine erwärmbare Wundauflage bekannt, die ein elektrisch leitfähiges textiles Heizelement und einen Kontrollschaltkreis zur Kontrolle der elektrisch leitfähigen Textilie aufweist. Das elektrisch leitfähige Textil kommt entweder direkt in Kontakt mit der Haut des Patienten oder ist der mit der Haut des Patienten in Kontakt kommenden Schicht benachbart angeordnet.

Die Druckschrift WO 94/15668 A1 beschreibt elektrische Stimulations-Körperpackung mit einer flexiblen Tasche die aus einem elektrisch isolierenden Tuch gebildet ist und einer Einrichtung zur Aufnahme eines Wärmeübertragungsmediums. Die Tasche weist eine erste Fläche mit einer Feuchtigkeitsbarriere zum Trennen eines gekühlten Wärmeübertragungsmediums von einem in Kontakt stehenden Körper auf und eine zweite Oberfläche mit einer Isoliereinrichtung zum Trennen eines erwärmten Wärmeübertragungsmediums von einem in Kontakt stehenden Körper, um Hautverbrennungen zu vermeiden. Die Einrichtung zum Anordnen und Drücken der ersten Oberfläche der flexiblen Tasche gegen einen Körperteil ist so ausgebildet, dass sie entweder die erste oder zweite Oberfläche der flexiblen Tasche gegen einen Körperteil drückt. Die Körperpackung weist ferner eine flexible elektrische Nerven- und Muskelstimulationselektrode auf, die an der flexiblen Tasche in einer Position befestigt ist, welche einen direkten Kontakt zwischen der Stimulationselektrode und dem Körperteil ermöglicht, wenn entweder die erste oder zweite Fläche der flexiblen Tasche gegen den Körperteil gedrückt wird, wobei ein Leitungsdraht elektrisch mit der flexiblen Nerven- und Muskel-Stimulationselektrode verbunden ist.

Mit der US 2003/0186608 A1 wird ein Gewebe mit schmerzlindernden Eigenschaften offenbart, das aus einem elektrisch leitfähigen Garn und einem elektrisch nicht leitfähigen Garn hergestellt wird. Das Gewebe kann in Textilprodukte wie Bandagen, Stützbändern und Kleidung eingearbeitet werden.

Mit der Druckschrift WO 2004/107816 A1 wird eine Vorrichtung offenbart, die zur topischen Verabreichung eines Wirkstoffs geeignet ist und ein atmungsaktives Heizelement umfasst. Bei dem atmungsaktiven Heizelement handelt es sich um ein metallisiertes Gewebe. Das flächige Heizelement ist auf eine Haut- oder Wundkontaktschicht aufgebracht und wird von einer Klebschicht überdeckt. Bei dem Heizelement handelt es sich um eine gewundene metallische Leiterbahn in einem geätzten Gewebe. Die Hautkontaktschicht kann einen mikroverkapselten Wirkstoff enthalten, der durch Hitzeaktivierung aus den dann schmelzenden Mikrokapseln freigesetzt und an die Haut abgegeben werden kann. Die gewundene metallische Leiterbahn als Heißelement führt jedoch zu einer Wärmeverteilung, die nicht homogen genug ist.

Elektrisch leitfähige Textilien mit dreidimensionaler Faserverteilung werden in der DE 20 2013 006 258 U1 als Vorrichtung zum Erzeugen von heizbaren Flächen zum gleichmäßigen Erwärmen von Kunststoffformen bei der Herstellung von thermo- oder duroplastischen Faserkunststoffverbundbauteilen vorgeschlagen.

Die WO 2005/044141 A2 beschreibt einen elektrisch erwärmbaren Wundverband.

Der vorliegenden Erfindung lag daher auch die Aufgabe zugrunde, ein Pflaster bereitzustellen, das zur Erzeugung von Wärme befähigt ist, wobei die Wärmeverteilung über der Fläche, insbesondere der Hautkontaktfläche des Pflasters, möglichst homogen ist.

Die Aufgabe wird entsprechend eines ersten Aspekts der Erfindung durch ein elektrisch erwärmbares Pflaster gemäß Anspruch 1.

Gemäß einem zweiten Aspekt betrifft die Erfindung ein Verfahren zur Herstellung von elektrisch erwärmbaren Pflastern gemäß Anspruch 8. Gemäß einem dritten Aspekt betrifft die Erfindung die Verwendung eines elektrisch leitfähigen, textilen Flächengebildes, gemäß Anspruch 10.

Gemäß einem weiteren Aspekt betrifft die Erfindung ein elektrisch erwärmbares Pflaster, gemäß einem der Ansprüche 1 bis 7, zur Verwendung für die lokale Wärmetherapie.

Gemäß einem weiteren Aspekt betrifft die Erfindung ein elektrisch erwärmbares Pflaster, gemäß einem der Ansprüche 1 bis 7 zur Verbesserung der Hautpermeation für einen in dem Pflaster enthaltenen Wirkstoff.

Ferner offenbart ist die Verwendung von Pflastern, umfassend eine haftklebende Hautkontaktschicht und ein elektrisch leitfähiges, textiles Flächengebilde, bei dem elektrisch leitfähige Fasern miteinander in Kontakt stehen, zur Verabreichung mindestens eines in dem Pflaster enthaltenen Wirkstoffs an und/oder über die Haut eines Säugetiers.

Ferner offenbart sind Verfahren zur Verabreichung von Wärme und/oder mindestens einem Wirkstoff an und/oder über die Haut eines Säugetiers mittels eines elektrisch erwärmbaren Pflasters, das eine haftklebende Hautkontaktschicht und eine elektrisch leitfähiges, textiles Flächengebilde umfasst, bei dem elektrisch leitfähige Fasern miteinander in Kontakt stehen. Unter einem Pflaster gemäß dem ersten Aspekt werden flächenförmige, flexible, vorzugsweise haftklebende Elemente verstanden, die an oder auf der Haut eines Säugetiers, vorzugsweise eines Menschen, angebracht werden können. Im Sinne der vorliegenden Offenbarung umfasst der Begriff "Pflaster" sowohl sogenannte Heftpflaster wie auch sogenannte Arzneipflaster. Heftpflaster sind klebende Textilbänder, die üblicherweise zum Fixieren von Verbänden oder Artikeln auf der Haut eines Patienten verwendet werden. Unter Arzneipflaster werden flächenförmige, flexible, vorzugsweise haftklebende Elemente verstanden, die mindestens einen pharmazeutischen Wirkstoff enthalten, der nach Applikation des Arzneipflasters aus diesem freigesetzt und an oder über die Haut des Patienten verabreicht wird.

Pflaster im Sinne der vorliegenden Offenbarung dienen nicht der Abdeckungvon Wunden, sondern werden auf intakter Haut befestigt. Der Begriff "Pflaster" im Sinne der vorliegenden Offenbarung umfasst somit keine vorgefertigten Wundschnellverbände, bei denen ein Stück Wundauflage mit einem Klebeband aus Gewebe oder Kunststoff verbunden ist.

Das Pflaster gemäß dem ersten Aspekt ist ein elektrisch erwärmbares Pflaster. Das bedeutet, dass das Pflaster Wärme erzeugen kann, wenn ein elektrischer Strom durch zumindest ein Teil oder Bauteil des Pflasters fließt.

Das Pflaster gemäß dem ersten Aspekt umfasst eine haftklebende Hautkontaktschicht und ein elektrisch leitfähiges, textiles Flächengebilde, bei dem elektrisch leitfähige Fasern miteinander in Kontakt stehen. Die haftklebende Hautkontaktschicht ist die Schicht des Pflasters, mit der es an oder auf der Haut eines Patienten befestigt wird. Die Hautkontaktschicht umfasst oder besteht aus einem hautverträglichen Haftklebstoff. Der hautverträgliche Haftklebstoff kann aus der Gruppe von Haftklebstoffen ausgewählt sein, die Polyacrylate, Polymethacrylate, Silicone, Polyisubutylene und deren Mischungen umfasst.

Das Pflaster umfasst ferner ein elektrisch leitfähiges, textiles Flächengebilde, bei dem elektrisch leitfähige Fasern miteinander in Kontakt stehen. Dieses Flächengebilde ist zur Erzeugung von Wärme befähigt, sofern es Teil eines elektrischen Stromkreises ist und von elektrischem Strom durchflossen wird. Das elektrisch leitfähige, textile Flächengebilde ist flexibel und kann sich somit der Kontur der Oberfläche anpassen, auf die das Pflaster geheftet wird, so dass eine möglichst gleichförmige Erwärmung des Hautareals gewährleistet werden kann und das Pflaster vom Patienten nicht als zu sehr störender Fremdkörper empfunden wird.

Erfindungsgemäß bildet das elektrisch leitfähige, textile Flächengebilde, bei dem elektrisch leitfähige Fasern miteinander in Kontakt stehen, die Rückschicht des Pflasters. Diese Ausführungsform stellt die einfachste Ausgestaltung des Pflasters dar, die vergleichsweise einfach und kostengünstig zu fertigen ist.

Bei einer alternativen nicht erfindungsgemäßen Ausführungsform umfasst das Pflaster eine haft- klebende Hautkontaktschicht, ein elektrisch leitfähiges, textiles Flächen- gebilde und zusätzlich eine Rückschicht. Bei dieser Ausführungsform stellt das elektrisch leitfähige, textile Flächengebilde, bei dem elektrisch leitfähige Fasern miteinander in Kontakt stehen, nicht die Rückschicht des Pflasters dar. Bei dieser Ausführungsform ist das elektrisch leitfähige, textile Flächengebilde eine zusätzliche Schicht. Diese Ausführungsform ist besonders vorteilhaft bei Ausführungsformen von Pflastern, die einen pharmazeutischen Wirkstoff erhalten, weil die Rückschicht so beschaffen gewählt werden kann, dass sie für den Wirkstoff undurchlässig ist.

Gemäß einer zusätzlichen und/oder alternativen nicht erfindungsgemäßen Ausführungsform ist die zusätzliche Rückschicht ein textiles Flächengebilde oder eine Polymerfolie, vorzugsweise eine wirkstoffundurchlässige Polymerfolie. Das textile Flächengebilde ist bei einer weiteren Ausführungsform ein Gewebe, Vlies oder Gewirke aus natürlichen Fasern, aus synthetischen Fasern oder aus einem Gemisch aus natürlichen und synthetischen Fasern. Gemäß einer anderen nicht erfindungsgemäßen Ausführungsform ist die Polymerfolie perforiert. Ein textiles Flächengebilde oder eine perforierte Polymerfolie als Rückschicht des Pflasters beeinträchtigen den Gasaustausch oder die Sauerstoffversorgung der Haut weniger als eine Rückschicht aus einer nicht perforierten Polymerfolie.

Bei einer zusätzlichen und/oder alternativen Ausführungsform weist das Pflaster eine Wärmeisolierschicht auf. Eine Wärmeisolierschicht sorgt dafür, dass die mit dem Pflaster erzeugte Wärme im Wesentlichen in Richtung Haut abgegeben wird. Die nach Applikation des Pflasters freie Oberfläche des Pflasters erwärmt sich weniger stark, was von dem Patienten als angenehm empfunden wird. Zudem wird weniger elektrische Energie benötigt, um eine vorgegebenen Temperatur zu halten. Somit kann eine gegebene Stromquelle länger genutzt werden oder es können Stromquellen mit geringerer Leistung verwendet werden, die üblicherweise auch kleiner und/oder leichter sind.

Die Wärmeisolierschicht ist bei einer nicht erfindungsgemäßen Ausführungsform des Pflasters auf der der Haut des Patienten abgewandten Fläche des elektrisch leitfähigen, textilen Flächengebildes angeordnet. Bei einer Ausgestaltung dieser nicht erfindungsgemäßen Ausführungsform ist die Rückschicht des Pflasters als Wärmeisolierschicht ausgebildet, insbesondere bei Ausführungsformen von Pflastern, bei denen das elektrisch leitfähige, textile Flächengebilde nicht die Rückschicht des Pflasters bildet. Bei einer alternativen nicht erfindungsgemäßen Ausgestaltung der Ausführungsform ist die Wärmeisolierschicht eine Schicht, die zusätzlich zu der Rückschicht vorhanden und somit zwischen dem elektrisch leitfähigen, textilen Flächengebilde und der Rückschicht angeordnet ist. Ein Pflaster gemäß dieser nicht erfindungsgemäßen Ausführungsform weist somit eine haftklebende Hautkontaktschicht, ein elektrisch leitfähiges, textiles Flächengebilde, eine Wärmeisolierschicht und eine Rückschicht auf.

Bei der Wärmeisolierschicht kann es sich beispielsweise um eine Schicht aus einem Polymerschaum handeln. Der Polymerschaum für die Wärmeisolierschicht kann ein offenporiger Schaum sein, also ein Polymerschaum, bei dem die Poren in offener Verbindung zueinander stehen, oder ein geschlossenporiger Schaum, bei den die einzelnen Poren nicht offen miteinander verbunden sind.

Bei einer zusätzlichen und/oder alternativen Ausführungsform weist das Pflaster eine ablösbare Schutzschicht auf, die die haftklebende Hautkontaktschicht des Pflasters vor dessen Anwendung abdeckt. Die ablösbare Schutzschicht muss vor der Anwendung des Pflasters von dessen haftklebender Fläche abgezogen werden.

Gemäß einer zusätzlichen und/oder alternativen nicht erfindungsgemäßen Ausführungsform ist das elektrisch leitfähige, textile Flächengebilde zwischen der haftklebenden Hautkontaktschicht und der Rückschicht angeordnet. Bei einer anderen nicht erfindungsgemäßen Ausführungsform liegt das elektrisch leitfähige, textile Flächengebilde in der haftklebenden Hautkontaktschicht eingebettet vor.

Bei dem elektrisch leitfähigen, textilen Flächengebilde handelt es sich um ein textiles Flächengebilde mit dreidimensionaler Faserverteilung. Das bedeutet, dass sich die Fasern in dem textilen Flächengebilde kreuzen oder überlappen. Dadurch stehen die elektrisch leitfähigen Fasern des textilen Flächengebildes in dem textilen Flächengebilde in Kontakt miteinander, so dass ein elektrischer Strom über die gesamte Fläche des textilen Flächengebildes fließen und sich das textile Flächengebilde möglichst gleichmäßig erwärmen kann. Der Begriff "miteinander in Kontakt stehen" umfasst sowohl das direkte einander Berühren elektrischer Fasern an ihren Kreuzungspunkten wie auch Ausgestaltungen, bei denen sich an den Kreuzungspunkten ein elektrisch leitfähiges Material zwischen zwei sich kreuzenden elektrisch leitfähigen Fasern des textilen Flächengebildes befindet.

Bei dem textilen Flächengebilde handelt es sich um ein Gewebe, ein Gewirke, ein Gestricke, ein Geflecht, ein bidirektionales oder multidirektionales Gelege, ein Filz oder um ein Vlies (Faservlies oder Spinnvlies) aus Fasern.

Gemäß einer bevorzugten Ausführungsform handelt es sich bei dem Gewebe um ein Gewebe in Leinwandbindung, Körperbindung oder Atlasbindung. Besonders bevorzugt handelt es sich bei dem Gewebe um ein 5-bindiges oder 8-bindiges Atlas. Der Vorteil des Atlas liegt einerseits darin, dass er besonders drapierfähig ist und andererseits derart orientiert im Pflaster angeordnet werden kann, dass eine möglichst hohe Zahl elektrisch leitfähiger Fasern möglichst nahe an der Fläche des Pflasters liegen, die mit der Haut des Patienten in Kontakt kommt.

In einer alternativen und/oder zusätzlichen Ausführungsform ist das elektrisch leitfähige, textile Flächengebilde aus der Gruppe von textilen Flächengebilden ausgewählt, die aus
a) ausschließlich aus elektrisch leitfähigen Fasern;
b) aus Mischungen von elektrisch leitfähigen und elektrisch nicht leitfähigen
   Fasern; und
c) aus einem oder mehreren elektrisch nicht leitfähigen textilen Flächengebilden, das/die mit klebrigen, elektrisch leitfähigen Binder(n) und elektrisch leitfähigen Partikeln, vorzugsweise Metallpartikeln, die dreidimensional und nachhaltig im textilen Flächengebilde fixiert sind, ausgerüstet ist/sind;
   besteht.

Unter "elektrisch leitfähigen Fasern" werden Fasern verstanden, die elektrischen Strom leiten können. Elektrisch leitfähige Fasern sind beispielsweise Fasern, die aus einem elektrisch leitfähigen Material bestehen, beispielsweise einem Metall, einer Legierung oder einem elektrisch leitfähigen Kunststoff. Unter dem Begriff "elektrisch leitfähige Fasern" werden aber auch Fasern verstanden, die mindestens einen elektrisch leitfähigen Kern umfassen, der von einem elektrisch nicht leitfähigen Material ummantelt ist. Der mindestens eine elektrisch leitfähige Kern der elektrisch leitfähigen Faser besteht aus einem elektrisch leitfähigen Material, beispielsweise einem Metall, einer Legierung oder einem elektrisch leitfähigen Kunststoff. Unter dem Begriff "elektrisch leitfähige Fasern" werden aber auch Fasern verstanden, die mindestens einen elektrisch nicht leitfähigen Kern aufweisen, der mit einem elektrisch leitfähigen Material ummantelt ist. Bei dem elektrisch leitfähigen Material für den elektrisch leitfähigen Mantel kann es sich um ein Metall, eine Legierung oder einen elektrisch leitfähigen Kunststoff handeln. Ein Beispiel für derartige Fasern sind mit Silber ummantelte Polyamidfasern.

Bei dem Pflaster gemäß dem ersten Aspekt der Erfindung handelt es sich um ein elektrisch erwärmbares Pflaster. Das bedeutet, dass durch Anlegen einer elektrischen Spannung an das elektrisch leitfähige, textile Flächengebilde des Pflasters und dem dadurch resultierenden Stromfluss Wärme erzeugt werden kann. Dabei ist die Temperatur, die mit dem elektrisch leitfähigen, textilen Flächengebilde erzeugbar ist, vorzugsweise gemessen an der Oberfläche des elektrisch leitfähigen, textilen Flächengebildes, bei einer gegebenen Spannung oder Stromstärke durch die Art und den Anteil der elektrisch leitfähigen Fasern in dem elektrisch leitfähigen, textilen Flächengebilde bzw. in der Mischung aus elektrisch leitfähigen oder metallummantelten Fasern und den nicht elektrisch leitfähigen Fasern einstellbar. Generell gilt: je geringer der Anteil an elektrisch leitfähigen Fasern und/oder Partikeln in dem elektrisch leitfähigen, textilen Flächengebilde, desto höher die zu erreichende Temperatur. Alternativ und/oder zusätzlich lässt sich der Grad der Erwärmung bei einem gegebenen elektrisch leitfähigen, textilen Flächengebilde über die Spannung und/oder die Stromstärke einstellen, die an das elektrisch leitfähige, textile Flächengebilde angelegt wird. Gemäß einer Ausführungsform liegt die Spannung, die während der Applikation des Pflasters an das elektrisch leitfähige, textile Flächengebilde angelegt wird bei mindestens 1,35 V und vorzugsweise bei weniger als 3,6 V, besonders bevorzugt bei weniger als 3,0 V. Bei Verwendung gebräuchlicher Batterien, beträgt die Spannung abhängig von der Batterie etwa 1,35 V, etwa 1,4 V, etwa 1,5 V, etwa 1,55 V oder etwa 3,0 V.

In einer Ausführungsform kann die Erwärmung des elektrisch leitfähigen, textilen Flächengebildes so eingestellt werden, dass die Temperatur - gemessen an der Oberfläche des elektrisch leitfähigen, textilen Flächengebildes - einen Wert von 50°C nicht übersteigt. Vorzugsweise ist die Erwärmung des elektrisch leitfähigen, textilen Flächengebildes so eingestellt, dass die Temperatur - gemessen an der Oberfläche des elektrisch leitfähigen, textilen Flächengebildes - einen Wert von 45°C, vorzugsweise einen Wert von 40°C, besonders bevorzugt einen Wert von 37°C, ganz besonders bevorzugt einen Wert von 35°C und ganz außerordentlich bevorzugt einen Wert von 32°C nicht übersteigt.

Der Flächen- bzw. Oberflächenwiderstand des elektrisch leitfähigen, textilen Flächengebildes und damit die Aufheizzeit bei gegebener Spannung und Heizfläche kann durch Wahl der elektrisch nicht leitfähigen Faserkomponente anwendungsbezogen eingestellt werden.

Je höher der Anteil an elektrisch nicht leitfähigen Fasern in der Mischung aus elektrisch leitfähigen oder metallummantelten Fasern und elektrisch nicht leitfähigen Fasern ist, desto länger beträgt die Aufheizzeit bei gegebener Spannung und Heizfläche, um eine bestimmte Oberflächentemperatur auf dem textilen Flächengebilde zu erreichen.

Gemäß einer zusätzlichen und/oder alternativen Ausführungsform liegt das elektrisch leitfähige, textile Flächengebilde in einer Harzschicht oder einer Polymerschicht eingebettet vor. Gemäß einer anderen zusätzlichen und/oder alternativen Ausführungsform ist das elektrisch leitfähige, textile Flächengebilde mit einem Harz oder einem Polymer imprägniert oder durchtränkt.

Gemäß einer zusätzlichen Ausführungsform weist das Harz oder das Polymer mindestens einen elektrisch leitfähigen Füllstoff auf. Der mindestens eine elektrisch leitfähige Füllstoff kann aus der Gruppe ausgewählt sein, die aus Graphit, Ruß, Kohlenstoffnanoröhrchen und Metallpartikeln besteht. Dadurch den mindestens einen elektrisch leitfähigen Füllstoff wird ein elektrisch leitfähiges Flächengebilde auf Basis eines Harzes oder Polymers hergestellt, mit dem eine besonders homogene Wärmeverteilung über die Fläche des (textilen oder textilverstärkten) Flächengebildes erreicht werden kann.

Bei einer zusätzlichen und/oder alternativen Ausführungsform ist das elektrisch leitfähige, textile Flächengebilde an geeigneten Stellen mit Elektroden versehen, über die eine Kontaktierung des elektrisch leitfähigen, textilen Flächengebildes mit einer Spannungsquelle und/oder einem elektronischen Bauteil zur Steuerung der Stromversorgung des elektrisch leitfähigen, textilen Flächengebildes erfolgen kann.

Das Kontaktieren des elektrisch leitfähigen, textilen Flächengebildes mit Elektroden kann durch Aufnähen, Verschweißen oder Ankleben der Elektroden mit/an dem elektrisch leitfähigen textilen Flächengebilde erfolgen. Die Kontaktierung sollte derart erfolgen, dass der Übergangswiderstand zwischen dem elektrischen Kontakt und dem elektrisch leitfähigen Textil möglichst gering ist, um Energieverluste und Stellen mit einer stärkeren Erwärmung sicher zu vermeiden. Vorzugsweise werden die Elektroden als parallele Leiter an dem elektrisch leitfähigen, textilen Flächengebilde angeordnet.

In einer zusätzlichen und/oder alternativen Ausführungsform umfasst das Pflaster eine Spannungsquelle, die mit dem elektrisch leitfähigen, textilen Flächengebilde elektrisch verbindbar ist. Vorzugsweise ist die Spannungsquelle eine Gleichstromquelle. Bei der Gleichstromquelle kann es sich um eine Batterie handeln. Besonders geeignet sind sogenannte Knopfzellen, also elektrochemische Zellen mit rundem Querschnitt, deren Gesamthöhe kleiner als der Gesamtdurchmesser ist. Beispiele für geeignete Knopfzellen sind:

| **Zellen-Typ** | **IEC-Bezeichnung** | **Beispiel** | **Spannung** |
|---|---|---|---|
| Quecksilberoxid-Zink-Zelle | MR | MR52 | 1,35 V |
| Zink-Luft-Zelle | PR | PR41 | 1,4 V |
| Alkali-Mangan-Zelle | LR | LR44, L1154 | 1,5 V |
| Silberoxid-Zink-Zelle | SR | SR44, SR1154 | 1,55 V |
| Lithium-Mangandioxid-Zelle | CR | CR2032 | 3,0 V |
| | | CR2450 | 3,0 V |
| Lithium-Kohlenstoffmonofluorid-Zelle | BR | BR2016 | 3,0 V |

In einer zusätzlichen und/oder alternativen Ausführungsform umfasst das Pflaster einen Schalter, mit dem der Stromkreis zwischen elektrisch leitfähigem, textilem Flächengebilde und Spannungsquelle geschlossen und/oder wieder unterbrochen werden kann. Bei dieser Ausführungsform kann das Pflaster nach seiner Applikation auf der Haut des Patienten zu beliebiger Zeit Wärme erzeugen beziehungsweise die Wärmeerzeugung bei Bedarf unterbrochen werden, ohne dass das Pflaster von der Haut entfernt werden muss.

Bei einer zusätzlichen und/oder alternativen Ausführungsform weist das Pflaster neben einer Spannungsquelle einen Isolierstreifen auf, der den Stromkreis zwischen Spannungsquelle und elektrisch leitfähigem, textilem Flächengebilde unterbricht. Dieser Isolierstreifen besteht aus einem elektrisch nicht leitfähigen Material, vorzugsweise einem elektrisch nicht leitfähigen Kunststoff. Der Isolierstreifen ist entfernbar angeordnet. Das bedeutet, dass der Isolierstreifen herausziehbar ist, ohne dass eine Abdeckung oder ähnliches geöffnet werden muss, und durch das Entfernen des Isolierstreifens der Stromkreis zwischen Spannungsquelle und elektrisch leitfähigem, textilen Flächengebilde geschlossen wird. Dieser Isolierstreifen kann unmittelbar vor der Anwendung des Pflasters entfernt werden.

In einer zusätzlichen und/oder alternativen Ausführungsform umfasst das Pflaster eine Ansteuer- und Regelelektronik, mit der die Temperatur des textilen Flächengebildes konstant gehalten werden kann.

In einer zusätzlichen und/oder alternativen Ausführungsform umfasst das Pflaster eine optische Funktionsanzeige. Die optische Funktionsanzeige dient dazu, dem Anwender anzuzeigen, dass das Pflaster ordnungsgemäß funktioniert. Bei der optischen Funktionsanzeige kann es sich beispielsweise um eine Leuchtdiode handeln, die leuchtet, sofern der Stromkreis zwischen Spannungsquelle und elektrisch leitfähigem, textilen Flächengebilde
geschlossen ist und elektrischer Strom fließt. Eine alternative und/oder zusätzliche optische Funktionsanzeige kann ein Temperaturmessstreifen sein, der die Temperatur, vorzugsweise die Temperatur an der Oberfläche des elektrisch leitfähigen, textilen Flächengebildes, anzeigt.

Das Pflaster enthält mindestens einen pharmazeutischen Wirkstoff. Einen pharmazeutischen Wirkstoff enthaltende Pflaster werden auch als transdermale therapeutische Systeme oder Wirkstoffpflaster bezeichnet. Es handelt sich hierbei um Vorrichtungen, die geeignet sind, einen Wirkstoff, vorzugsweise einen pharmazeutischen Wirkstoff, über einen längeren Zeitraum hinweg mit konstanter oder zumindest annähernd konstanter Rate an die und über die Haut des Nutzers/Patienten abzugeben.

Bei einer Ausführungsform des wirkstoffhaltigen Pflasters wird die haftklebende Hautkontaktschicht von einer haftklebenden Polymermatrix gebildet, die auch den mindestens einen Wirkstoff oder mindestens einen der Wirkstoffe enthält. Bei einer zusätzlichen und/oder alternativen Ausführungsform ist die haftklebende Hautkontaktschicht eine separate Haftkleberschicht, die zwar auch den mindestens einen Wirkstoff enthalten kann, jedoch nicht als Wirkstoffreservoir des Pflasters fungiert. Die separate Haftkleberschicht ist auf zumindest einen Bereich der hautseitigen Fläche eines zusätzlich vorhandenen Wirkstoffreservoirs aufgebracht.

Bei dem mindestens einen Wirkstoffreservoir eines transdermalen therapeutischen Systems handelt es sich entweder um eine Polymermatrix, in der der mindestens eine Wirkstoff enthalten ist, oder um ein beutel- förmiges Reservoir, welches von einer Hülle begrenzt wird und eine im Wesentlichen flüssige Wirkstoffzubereitung enthält. Der Begriff "flüssig" umfasst auch dünnflüssige, dickflüssige sowie gelartige Zubereitungen. Die Hülle des beutelförmigen Reservoirs weist zumindest auf seiner der Haut
zuzuwendenden Seite eine semipermeable Membran auf, über die der in dem Reservoir enthaltene Wirkstoff abgegeben werden kann und die ggf. eine die Freisetzungsrate des Wirkstoffs kontrollierende Funktion hat. Sofern der mindestens eine Wirkstoff in einer Polymermatrix des transdermalen therapeutischen Systems enthalten ist, ist diese Polymermatrix als Wirkstoffreservoir anzusehen.

Das wirkstoffhaltige Pflaster enthält mindestens einen Wirkstoff, vorzugsweise mindestens einen pharmazeutischen Wirkstoff. Bei dem mindestens einen pharmazeutischen Wirkstoff kann es sich um einen beliebigen, transdermal verabreichbaren pharmazeutischen Wirkstoff handeln. Beispielsweise kann der mindestens eine pharmazeutische Wirkstoff aus der Gruppe ausgewählt sein, die nichtsteroidale Antirheumatika (NSAID's) Anticholinergika, Parasympatholytika, Antimykotika, MAO-B-Inhibitoren, Serotonin-Antagonisten, Alpha2-Rezeptor-Agonisten, Photosensibilatoren, Hormone und/oder Proteine umfasst.

Die nichtsteroidalen Antirheumatika (NSAR oder NSA) - auch nichtsteroidales Antiphlogistikum (NSAP) oder NSAID (non-steroidal antiinflammatory drugs) - sind Schmerzmittel (Nichtopioid-Analgetika), die ihrer entzündungshemmenden (antiphlogistischen) Wirkung wegen symptombezogen auch zur Rheumatherapie eingesetzt werden. Gemäß einer Ausführungsform ist das nichtsteroidale Antirheumatikum aus der Gruppe ausgewählt, die aus Anthranilsäurederivaten, beispielsweise Mefenaminsäure, Flufenaminsäure, Etofenamat und Meclofenaminsäure, selektiven COX-2-Inhibitoren, beispielsweise Celecoxib, Etoricoxib, Rofecoxib, Lumiracoxib und Valdecoxib, Essigsäurederivaten und Arylessigsäurederivaten, beispielsweise Aceclofenac, Acemetamin, Bufexamac, Diclofenac, Etodolac, Indometamin und Ketorolac, Oxicame, beispielsweise Lornoxicam, Meolxicam, Piroxicam und Tenoxicam, Propionsäurederivate, beispielsweise Ibuprofen, Dexibuprofen, Naproxen, Ketoprofen, Dexketoprofen, Flurbiprofen, Benoxaprofen und Tiaprofensäure, Salicylate, beispielsweise Acetylsalicylsäure, Calciumcarbasalat, Lysinacetylsalicylat und Salicylsäure, sowie Nabumeton und Nimesulid besteht.

Der mindestens eine pharmazeutische Wirkstoff liegt in Form seiner freien Base und/oder mindestens eines seiner pharmazeutisch annehmbaren Salze vor. Der Begriff "pharmazeutisch annehmbares Salz" schließt auch pharmazeutisch annehmbare Säureadditionssalze des Wirkstoffs mit ein. Sofern es sich bei dem mindestens einen Wirkstoff um eine chirale Substanz handelt, liegt der Wirkstoff entweder in Form eines Racemats oder in Form seines pharmazeutisch wirksamen Enantiomers in dem transdermalen therapeutischen System vor.

In einer zusätzlichen und/oder alternativen Ausführungsform umfasst das transdermale therapeutische System mindestens eine Permeationsenhancer, der die Permeation des mindestens einen Wirkstoffs durch die Haut des Patienten verbessert.

Bei einer Ausführungsform weist das wirkstoffhaltige Pflaster eine wirkstoffundurchlässige Rückschicht auf. Die wirkstoffundurchlässige Rückschicht besteht üblicherweise aus einer für den im wirkstoffhaltigen Pflaster enthaltenen Wirkstoff undurchlässige Polymerfolie. Die wirkstoffundurchlässige Rückschicht bietet einen Schutz für die wirkstoffhaltige Schicht. Die Rückschicht verhindert zudem, dass der Patient oder eine andere Person in unbeabsichtigter Weise mit dem im Pflaster enthaltenen Wirkstoff in Kontakt kommt, und stellt sicher, dass der Wirkstoff gerichtet an die Haut des Patienten freigesetzt wird.

Transdermale therapeutische Systeme sind komplexe Darreichungsformen, bei denen einander zum Teil entgegenstehende Anforderungen erfüllt werden müssen. Beispielsweise muss der Wirkstoffgehalt in einem transdermalen therapeutischen System groß genug sein, um eine für den therapeutischen Nutzen erforderliche Menge an Wirkstoff pro Zeiteinheit über einen längeren Zeitraum hinweg abgeben zu können. Hierbei sind der Größe eines transdermalen therapeutischen Systems jedoch Grenzen gesetzt, insbesondere bedingt durch Erfordernisse bei der Herstellung, Handhabung und Patientencompliance.

Um die transdermale Verabreichung eines Wirkstoffs zu verbessern, können beispielsweise so genannte Permeationsenhancer verwendet werden. Hierbei handelt es sich um Substanzen, die im transdermalen therapeutischen System enthalten sind und die Haut für den ebenfalls im Pflaster enthaltenen Wirkstoff durchlässiger machen. Gemäß einer Ausführungsform umfasst das wirkstoffhaltige Pflaster zusätzlich mindestens einen Permeationsenhancer und/oder mindestens einen pharmazeutisch annehmbaren Hilfsstoff.

Allerdings kann die Verwendung von Permeationsenhancern mit unerwünschten Nebenwirkungen wie Hautreizungen einhergehen. Ohne eine Verwendung von Permeationsenhancern und/oder anderen pharmazeutisch annehmbaren Hilfsstoffen im Pflaster auszuschließen, ermöglicht das elektrisch erwärmbare Pflaster eine Verbesserung der Hautpermeation eines im Pflaster enthaltenen Wirkstoffs, indem das Hautareal, in dem der Wirkstoff perkutan verabreicht werden soll, mit Hilfe des elektrisch erwärmbaren Pflasters erwärmt wird. Dadurch kann gegebenenfalls der Gehalt an Permeationsenhancer im Pflaster geringer als bei einem vergleichbaren, nicht erwärmbaren Pflaster sein, um die gleiche Permeationsrate zu erreichen.

In einer nicht erfindungsgemäßen Ausführungsform ist das elektrisch leitfähige, textile Flächengebilde zwischen der wirkstoffhaltigen Schicht und der wirkstoffundurchlässigen Rückschicht des wirkstoffhaltigen Pflasters angeordnet.

In einer alternativen Ausführungsform wird das elektrisch leitfähige textile Flächengebilde durch eine wirkstoffundurchlässige Schicht von der wirkstoffhaltigen Schicht getrennt.

Gemäß dem zweiten Aspekt betrifft die Erfindung ein Verfahren zur Herstellung von elektrisch erwärmbaren Pflastern wie vorstehend beschrieben.

Bei einer Ausführungsform des Verfahrens umfasst das Herstellungsverfahren zumindest die folgenden Schritte:
- das Bereitstellen eines elektrisch leitfähigen, textilen Flächengebildes, wobei das elektrisch leitfähige, textile Flächengebilde ein Gewebe, ein Gewirke, ein Gestricke, ein Geflecht, ein bidirektionales oder multidirektionales Gelege, ein Filz oder ein Vlies aus Fasern ist, bei dem elektrisch leitfähige Fasern miteinander in Kontakt stehen,
- das Laminieren des elektrisch leitfähigen, textilen Flächengebildes auf eine haftklebende Schicht,
- das Vereinzeln der individuellen Pflaster, und
- das Anbringen der Kontakte auf die nicht laminierte Seite des elektrisch leitfähigen, textilen Flächengebildes.

Bei einer zusätzlichen und/oder alternativen Ausführungsform umfasst das Verfahren auch Laminieren mindestens einer weiteren Schicht. Bei einer nicht erfindungsgemäßen Ausgestaltung dieser Ausführungsform handelt es sich bei der mindestens einen weiteren Schicht um eine Polymerfolie, eine perforierte Polymerfolie oder ein textiles Flächengebilde wie ein Gewebe, Gewirke, Gelege oder Vlies, oder um eine Schicht aus einem offenporigen Schaumstoff oder aus einem geschlossenporigen Schaumstoff handeln, die vorzugsweise auf der

Fläche des elektrisch leitfähigen, textilen Flächengebildes aufgebracht wird, die der haftklebenden Schicht, welche die Hautkontaktschicht des Pflaster darstellt, abgewandt ist. Bei einer anderen und/oder zusätzlichen Ausgestaltung handelt es sich bei der mindestens einen weiteren Schicht um eine Polymerschicht, die beispielsweise als wirkstoffhaltiges Reservoir zwischen der haftklebenden Schicht und dem elektrisch leitfähigen, textilen Flächengebilde angeordnet wird.

Bei einer zusätzlichen und/oder alternativen Ausführungsform umfasst das Verfahren das Anbringen einer Spannungsquelle und/oder eines Ein-/Ausschalters und/oder eines Isolierstreifens.

Bei einer Ausführungsform des Verfahrens weist die haftklebende Kleberschicht keinen pharmazeutischen Wirkstoff auf. Bei einer alternativen Ausführungsform weist die haftklebende Kleberschicht mindestens einen pharmazeutischen Wirkstoff und optional mindestens einen Permeationsenhancer und/oder mindestens einen pharmazeutisch annehmbaren Hilfsstoff auf.

Bei einer zusätzlichen und/oder alternativen Ausführungsform ist die haftklebende Kleberschicht vor dem Laminieren des elektrisch leitfähigen, textilen Flächengebildes bereits auf eine Polymerfolie aufgebracht, die beispielsweise die abziehbare Schutzfolie des fertigen Pflasters darstellt.

Das Verfahren zur Herstellung der Pflaster umfasst üblicherweise das Bereitstellen der einzelnen Lagen oder Schichten des Pflasters vor Ihrem Zusammenfügen in Form von Rollen oder Bahnware. Die unterschiedlichen Bahnen werden im Herstellungsverfahren aufeinander gelegt und miteinander zu einem Laminat verbunden. Das Vereinzeln der Pflaster aus dem bahnförmigen Laminat kann durch Ausstanzen oder durch Schneiden erfolgen.

Gemäß dem dritten Aspekt betrifft die Erfindung die Verwendung eines elektrisch leitfähigen, textilen Flächengebildes, bei dem elektrisch leitfähige Fasern miteinander in Kontakt stehen, zur Herstellung der vorstehend beschriebenen elektrisch erwärmbaren Pflastern.

Durch die Verwendung eines elektrisch leitfähigen, textilen Flächengebildes als Heizelement bei einem Pflaster kann eine homogene Wärmeverteilung erreicht werden, so dass es trotz niedriger Spannungen, eines geringen Abstands des Heizelements zur Haut des Patienten, und gegebenenfalls nur geringfügigen Erhöhung der Temperatur auf eine Temperatur, die nur wenige Grad über die Temperatur an der Hautoberfläche des Patienten liegt, zu keinen unerwünschten Inhomogenitäten bei der Temperaturverteilung über die Fläche des Pflasters kommt. Dadurch kann eine besonders gleichmäßige Erwärmung der Haut und ein besonders guter Therapieerfolg erreicht werden.

Gemäß einem der weiteren Aspekte betrifft die Erfindung das vorstehend beschriebene elektrisch erwärmbare Pflaster, zur Verwendung für die lokale Wärmetherapie. Die Offenbarung erstreckt sich somit auch auf Verfahren zur lokalen Wärmetherapie, bei der eines der vorstehend beschriebenen Pflaster verwendet wird, vorzugsweise ein wirkstofffreies Pflaster, das eine haftklebende Hautkontaktfläche und ein elektrisch leitfähiges, textiles Flächengebilde umfasst, bei dem elektrisch leitfähige Fasern miteinander in Kontakt stehen.

Gemäß einem anderen der weiteren Aspekte betrifft die Offenbarung die Verwendung der vorstehend beschriebenen Pflaster, die eine haftklebende Hautkontaktschicht und ein elektrisch leitfähiges, textiles Flächengebilde umfassen, bei dem elektrisch leitfähige Fasern miteinander in Kontakt stehen, zur Verbesserung der Hautpermeation eines in dem Pflaster enthaltenen Wirkstoffs. Die Offenbarung erstreckt sich somit auch auf Verfahren zur Erhöhung der Hautpermeation für einen Wirkstoff, vorzugsweise für einen pharmazeutischen Wirkstoff, bei dem ein Pflaster gemäß dem ersten Aspekt verwendet wird, das eine haftklebende Hautkontaktschicht, ein elektrisch leitfähiges, textiles Flächengebilde, bei dem elektrisch leitfähige Fasern miteinander in Kontakt stehen, sowie einen Wirkstoff umfasst.

Gemäß einem anderen der weiteren Aspekte betrifft die Offenbarung die Verwendung von Pflastern wie vorstehend beschrieben, die eine haftklebende Hautkontaktschicht, ein elektrisch leitfähiges, textiles Flächengebilde, bei dem elektrisch leitfähige Fasern miteinander in Kontakt stehen, sowie mindestens einen Wirkstoff umfassen, zur Verabreichung des mindestens einen Wirkstoffs an und/oder über die Haut eines Säugetiers.

Die Offenbarung erstreckt sich somit auch auf Verfahren zur transdermalen Verabreichung eines Wirkstoffs, vorzugsweise eines pharmazeutischen Wirkstoffs, bei denen ein den Wirkstoff enthaltendes, elektrisch erwärmbares Pflaster, das ein elektrisch leitfähiges, textiles Flächengebilde umfasst, bei dem elektrisch leitfähige Fasern miteinander in Kontakt stehen, und den Wirkstoff enthält, auf oder an der Haut eines Patienten befestigt wird.

Bei der Verwendung der erfindungsgemäßen Pflaster fungiert das textile Flächengebilde als Heizelement mit dem das Pflaster und das von dem Pflaster bedeckte Hautareal des Patienten erwärmt wird. Bei der Verwendung kommt es auf die Nutzung der Wärme an, nicht jedoch auf die Wirkung des für die Erwärmung des textilen Flächengebildes erforderlichen elektrischen Stroms an sich auf den Körper oder das von dem Pflaster überdeckten Körperareals. Insofern unterscheidet sich die erfindungsgemäße Verwendung der erfindungsgemäßen Pflaster von der Interferenzstrom-Regulationstherapie, bei der die Heilung von Wunden, beispielsweise Verletzungen des Epithels oder auch Operationswunden innerhalb des Körpers eines Patienten, mittels Elektrostimulation in Kombination mit einer konventionellen Wundversorgung gegenüber einer Standardwundversorgung beschleunigt wird.

Bei Verwendungen und/oder Verfahren nach den weiteren Aspekten der Offenbarung wird der Stromkreis zwischen einer Spannungsquelle und dem elektrisch leitfähigen, textilen Flächengebilde geschlossen, unmittelbar bevor oder nachdem das Pflaster an oder auf der Haut eines Säugetiers, vorzugsweise eines Menschen, befestigt wird. Durch Schließen des Stromkreises fließt elektrischer Strom durch das elektrisch leitfähige, textile Flächengebilde, welches sich in der Folge aufgrund der angelegten Spannung und seines Widerstands erwärmt. Die Erwärmung hat eine therapeutische Wirkung oder führt zu einer Erhöhung der Hautpermeation eines in dem Pflaster gegebenenfalls enthaltenen Wirkstoffs.

### Ausführungsbeispiele

### Beispiel 1: Ibuprofen-Pflaster mit elektrisch heizbarer Rückschicht

174,32 g Durotak 387-2353 (Feststoffgehalt 37%) wurden mit 8,07 g Ethylacetat verdünnt und 31,6 g einer Lösung von 10% Kaliumhydroxid in Methanol zugegeben. Nach vollständiger Vermischung wurde 16,63 g Ölsäure zugegeben. Anschließend wurden 1,2 g Aluminiumacetylacetonat und 1 g Acetylaceton zugegeben. Nachdem alle Bestandteile gut miteinander vermischt waren, wurden 16,63 g Ibuprofen (Racemat) zugegeben. Die Masse wurde bei Raumtemperatur bis zur vollständigen
Lösung aller Feststoffe gerührt. Die Klebermasse wurde anschließend mittels einer Rakel auf eine silikonisierte PET-Folie 100 µm derart ausgestrichen, dass ein Trockengewicht von 60 g/m² erreicht wurde. Die Lösemittel wurden bei ca. 80°C verdampft. Anschließend wurde das Laminat mit dem heizbaren Fasermaterial abgedeckt, bei dem es sich um ein Spinnvlies mit elektrisch leitfähigen Fasern handelte.

### Beispiel 2: Diclofenac-Pflaster mit elektrisch heizbarer Rückschicht

94,92 g Durotak 387-2287 (Feststoffgehalt 50%) wurden mit einer Lösung von 0,72 g Aluminiumacetylacetonat in 13 g Ethylacateat vermischt. Zu dieser Masse wurden 0,12 g α-Tocopherol in 0,4 g Ethylacetat gelöst gegeben und homogen verrührt. 2,4 g Diclofenac-Na wurden in 5,4 g Methanol gelöst und zu der Masse gegeben. Die Masse wurde bei Raumtemperatur bis zur vollständigen Lösung aller Feststoffe gerührt. Die Klebermasse wurde mittels einer Rakel auf eine silikonisierte PET-Folie 100 µm derart ausgestrichen, dass ein Trockengewicht von 80 g/m² erreicht wurde. Die Lösemittel werden bei ca. 75°C verdampft. Anschließend wurde das Laminat mit dem heizbaren Fasermaterial abgedeckt, bei dem es sich um ein Spinnvlies mit elektrisch leitfähigen Fasern handelte.

### Beispiel 3: Diclofenac-Pflaster mit elektrisch heizbarer Rückschicht 118,57 g

Durotak 387-2051 (Feststoffgehalt 50%) wurden mit 24 g einer Lösung aus 2,4 g Kaliumhydroxid in 21,6 g Methanol neutralisiert. 90,66 g der so neutralisierten Kleberlösung wurden mit 2,26 g einer Lösung von 0,3 g Aluminiumacetylacetonate in 1,75 g Methanol und 0,3 g Ethylacetat vermischt. 7,5 g Ölsäure und 0,25 g α-Tocopherol wurden hinzugefügt und bis zum vollständigen Lösen gerührt. Dazu wurden 6,38 g einer Lösung aus 2,0 g Diclofenac-Na in 4,38 g Methanol gegeben. Die Masse wurde bei Raumtemperatur bis zur vollständigen Lösung aller Feststoffe gerührt. Die Klebermasse wurde mittels einer Rakel auf eine silikonisierte PET-Folie 100 µm derart ausgestrichen, dass ein Trockengewicht von 80 g/m² erreicht wurde. Die Lösemittel wurden zunächst für ca. 10 Minuten bei Raumtemperatur abgelüftet und anschließend bei ca. 60°C verdampft. Anschließend wird das Laminat mit dem heizbaren Fasermaterial abgedeckt, bei dem es sich um ein Spinnvlies mit elektrisch leitfähigen Fasern handelte.

### Beispiel 4: Durchführung der Hautpermeationsexperimente

Für die Durchführung der in-vitro Permeationsversuche wurde eine modifiziert Diffusionszelle nach Keshary-Chien verwendet. Die Zelle besteht aus 2 horizontal geteilten Bereichen, dem Donorbereich und dem Akzeptorbereich. Die Temperatur der Diffusionszelle wurde mittels Wasserbad bei 32°C gehalten. Mittels eines Magnetrührers wurde das Akzeptormedium (Phosphatpuffer, pH 5,5) während des Versuchs ständig gerührt.

Die Haut wurde zwischen die beiden Bereiche eingeklemmt, wobei das Stratum corneum der Haut nach oben zum Donorbereich zeigte. Das Pflaster wurde mit der Klebeschicht zum Stratum corneum der Haut hin auf der Hautprobe positioniert.

An den spezifizierten Entnahmezeitpunkten wurde das Akzeptormedium vollständig aus dem Akzeptorbereich entfernt und für die spätere quantitative Bestimmung des darin gelösten Wirkstoffs aufbewahrt. Anschließend wurde die gleiche Menge frischen Akzeptormediums eingefüllt. Damit wurde sichergestellt, dass die Permeationskinetik nicht durch ein eventuelles Erreichen der Sättigungslöslichkeit im Akzeptormedium beeinflusst wird.

Für alle Hautpermeationsexperimente wurde dermatomisierte Humanhaut (800 µm) verwendet. Die Oberfläche der Öffnung der Zelle betrug 1,54 cm²

Die quantitative Bestimmung der in das Akzeptormedium permeierten Wirkstoffe erfolgte mittel Hochleistungsflüssigchromatographie (HPLC).

Zur Analyse von Diclofenac wurde als mobile Phase eine Mischung aus Acetonitril und 0,025 m KH₂PO₄ (50:50, v/v) verwendet. Der pH-Wert wurde auf 3,0 eingestellt. Als stationäre Phase wurde eine 150 x 4,6 mm Trennsäule, gepackt mit Zorbax SB C8 80 A 5 µm, verwendet. Die Flussrate lag bei 1,5 ml/min, die Säulentemperatur bei 30 °C. Das Injektionsvolumen betrug 50 µl, die Detektorwellenlänge war auf 225 nm eingestellt.

Für die Analyse von Ibuprofen wurde eine 150 x 4,0 mm Trennsäule gepackt mit Novapack C18 5 µm verwendet. Als mobile Phase wurde eine Mischung Acetonitril : Wasser: Tetramethylammoniumhydroxid im Mischungsverhältnis von 55 : 45 : 1,5 verwendet. Der pH-Wert wurde auf 3,0 eingestellt. Die Flussrate lag bei 1,0 ml/min, die Säulentemperatur bei 25 °C. Das Injektionsvolumen betrug 50 µl, die Detektorwellenlänge war auf 214 nm eingestellt.

Die Berechnung erfolgte über die Externe Standard Methode unter Verwendung von zertifizierten Referenzsubstanzen.

Die Pflaster wurden so groß hergestellt, dass ihre Ränder jeweils deutlich aus den Diffusionszellen herausragten. Die Kontaktierung der heizbaren Rückschicht erfolgte durch aufgenähte elektrisch leitfähige Gewebebahnen. An diese wurde mittels Krokodilklemmen eine Gleichspannung (POWER SUPPLY HM 7042-5, Fa. HAMEG) angelegt. Zur Erzielung einer Oberflächentemperatur von 42 °C wurde die Spannung auf 3V eingestellt.

Um 50°C Oberflächentemperatur zu erreichen wurde die Spannung auf 6 V einstellt. Die resultierende Stromstärke lag bei ca. 200 mA für 3 V und 42 °C bzw. bei ca. 50 mA für 6 V und 50 °C.

Als Referenz erfolgte eine parallele Messung ohne Strombeaufschlagung.

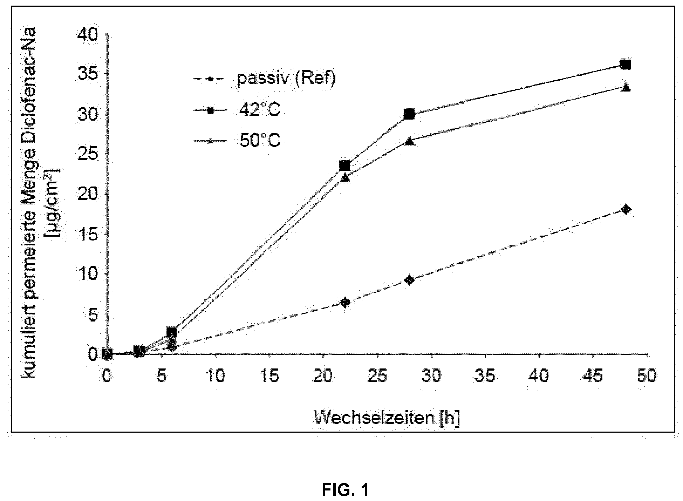

Die Ergebnisse der Hautpermeationsexperimente für das in Beispiel 2 beschriebene Diclofenac- Pflaster sind in Fig. 1 graphisch dargestellt. Aus dieser Darstellung wird ersichtlich, dass eine Erwärmung der Rückschicht des Diclofenac- Pflaster auf eine Temperatur von etwa 42 °C oder etwa 50°C zu einer deutlich verbesserten Permeation des in dem Pflaster enthaltenen Diclofenac über die Haut führt, verglichen mit der als "passiv" bezeichneten Referenz, bei der keine Gleichspannung angelegt wurde.

## Patentansprüche

1. Ein elektrisch erwärmbares Pflaster umfassend eine haftklebende Hautkontaktschicht und ein elektrisch leitfähiges, textiles Flächengebilde, wobei das elektrisch leitfähige, textile Flächengebilde mit elektrischen Kontakten versehen ist und wobei das elektrisch leitfähige, textile Flächengebilde ein Gewebe, ein Gewirke, ein Gestricke, ein Geflecht, ein bidirektionales oder multidirektionales Gelege, ein Filz oder ein Vlies aus Fasern ist, bei dem elektrisch leitfähige Fasern miteinander in Kontakt stehen, **dadurch gekennzeichnet, dass** das elektrisch leitfähige textile Flächengebilde die Rückschicht des Pflasters bildet und wobei das elektrisch erwärmbare Pflaster mindestens einen pharmazeutischen Wirkstoff enthält.

2. Das Pflaster gemäß Anspruch 1. **dadurch gekennzeichnet, dass** das textile Flächengebilde aus der Gruppe von textilen Flächengebilden ausgewählt, die
a) aus ausschließlich elektrisch leitfähigen Fasern, vorzugsweise metallischen Fasern;
b) aus Mischungen von elektrisch leitfähigen Fasern und elektrisch nicht leitfähigen Fasern; und/oder
c) aus einem oder mehreren elektrisch nicht leitfähigen textilen Flächengebilden, das/die mit klebrigen, elektrisch leitfähigen Binder(n) und elektrisch leitfähigen Partikeln, vorzugsweise Metallpartikeln, die dreidimensional und nachhaltig im textilen Flächengebilde fixiert sind, ausgerüstet ist/sind;
besteht.

3. Das Pflaster gemäß Anspruch 1 oder 2, **dadurch gekennzeichnet, dass** das elektrisch leitfähige Flächengebilde in einer Harzschicht oder Polymerschicht eingebettet vorliegt.

4. Das Pflaster gemäß Anspruch 3, **dadurch gekennzeichnet, dass** die Harzschicht oder Polymerschicht mindestens einen elektrisch leitfähigen Füllstoff aufweist.

5. Das Pflaster gemäß einem der vorhergehenden Ansprüche, **dadurch gekennzeichnet, dass** es eine Spannungsquelle aufweist, vorzugsweise eine Gleichstromquelle.

6. Das Pflaster gemäß einem der vorhergehenden Ansprüche, **dadurch gekennzeichnet, dass** es eine Ansteuer- und Regelelektronik aufweist, mit der die Temperatur des textilen Flächengebildes konstant gehalten werden kann.

7. Das Pflaster gemäß einem der vorhergehenden Ansprüche, **dadurch gekennzeichnet, dass** der pharmazeutische Wirkstoff ausgewählt ist aus der Gruppe, die nichtsteroidale Antirheumatika (NSAID's) Anticholinergika, Parasympatholytika, Antimykotika, MAO-B-Inhibitoren, Serotonin-Antagonisten, Alpha2-Rezeptor-Agonisten, Photosensibilatoren, Hormone und/oder Proteine umfasst.

8. Ein Verfahren zur Herstellung eines elektrisch beheizbaren Pflasters gemäß einem der Ansprüche 1 bis 7, umfassend die Schritte:
- das Bereitstellen eines elektrisch leitfähigen, textilen Flächengebildes, wobei das elektrisch leitfähige, textile Flächengebilde ein Gewebe, ein Gewirke, ein Gestricke, ein Geflecht, ein bidirektionales oder multidirektionales Gelege, ein Filz oder ein Vlies aus Fasern ist, bei dem elektrisch leitfähige Fasern miteinander in Kontakt stehen,
- das Laminieren des elektrisch leitfähigen, textilen Flächengebildes auf eine haftklebende Hautkontakt Schicht,
- das Vereinzeln der individuellen Pflaster, und
- das Anbringen der Kontakte auf die nicht laminierte Seite des elektrisch leitfähigen, textilen Flächengebildes.

9. Das Verfahren gemäß Anspruch 8, ferner umfassend das Versehen des Laminats mit mindestens einer weiteren Schicht.

10. Verwendung eines elektrisch leitfähigen, textilen Flächengebildes wobei das elektrisch leitfähige, textile Flächengebilde ein Gewebe, ein Gewirke, ein Gestricke, ein Geflecht, ein bidirektionales oder multidirektionales Gelege, ein Filz oder ein Vlies aus Fasern ist, bei dem elektrisch leitfähige Fasern miteinander in Kontakt stehen wobei das elektrisch leitfähige, textile Flächengebilde mit elektrischen Kontakten versehen ist, zur Herstellung von elektrisch erwärmbaren Pflastern gemäß einem der Ansprüche 1 bis 7.

11. Elektrisch erwärmbares Pflaster gemäß einem der Ansprüche 1 bis 7 zur Verwendung in der lokalen Wärmetherapie, zur Erhöhung der Hautpermeation für einen pharmazeutischen Wirkstoff, vorzugsweise für einen im Pflaster enthaltenen pharmazeutischen Wirkstoff, und/oder der transdermalen Verabreichung eines Wirkstoffs, vorzugsweise eines im Pflaster enthaltenen pharmazeutischen Wirkstoffs.

## Claims

1. An electrically heatable patch comprising a pressure-sensitive adhesive skin contact layer and an electrically conductive textile fabric, wherein the electrically conductive textile fabric is provided with electrical contacts and wherein the electrically conductive textile fabric is a woven fabric, a warp-knitted fabric, a knitted fabric, a braid, a bidirectional or multidirectional laid fabric, a felt or a non-woven fabric made of fibres, in which electrically conductive fibres are in contact with each other, **characterised in that** the electrically conductive textile fabric forms the backing layer of the patch and wherein the electrically heatable patch contains at least one pharmaceutical active ingredient.

2. The patch according to Claim 1, **characterised in that** the textile fabric is selected from the group of textile fabrics which consist
a) of exclusively electrically conductive fibres, preferably metallic fibres;
b) of mixtures of electrically conductive fibres and non-electrically conductive fibres; and/or
c) of one or more non-electrically conductive textile fabrics which is/are equipped with sticky, electrically conductive binder(s) and electrically conductive particles, preferably metal particles, which are fixed three-dimensionally and lastingly in the textile fabric.

3. The patch according to Claim 1 or Claim 2, **characterised in that** the electrically conductive fabric is present embedded in a resin layer or polymer layer.

4. The patch according to Claim 3, **characterised in that** the resin layer or polymer layer has at least one electrically conductive filler.

5. The patch according to one of the preceding claims, **characterised in that** it has a voltage source, preferably a direct current source.

6. The patch according to one of the preceding claims, **characterised in that** it has control electronics with which the temperature of the textile fabric can be kept constant.

7. The patch according to one of the preceding claims, **characterised in that** the pharmaceutical active ingredient is selected from the group comprising non-steroidal antirheumatics (NSAIDs), anticholinergics, parasympatholytics, antimycotics, MAO-B inhibitors, serotonin antagonists, alpha2-receptor agonists, photosensitisers, hormones and/or proteins.

8. A method for producing an electrically heatable patch according to one of Claims 1 to 7, comprising the steps:
- providing an electrically conductive textile fabric, wherein the electrically conductive textile fabric is a woven fabric, a warp-knitted fabric, a knitted fabric, a braid, a bidirectional or multidirectional laid fabric, a felt or a non-woven fabric made of fibres, in which electrically conductive fibres are in contact with each other,
- laminating the electrically conductive textile fabric to a pressure-sensitive adhesive skin contact layer,
- separating the individual patches, and
- attaching the contacts to the non-laminated side of the electrically conductive textile fabric.

9. The process according to Claim 8, further comprising providing the laminate with at least one further layer.

10. Use of an electrically conductive textile fabric, wherein the electrically conductive textile fabric is a woven fabric, a warp-knitted fabric, a knitted fabric, a braid, a bidirectional or multidirectional laid fabric, a felt or a non-woven fabric made of fibres, in which electrically conductive fibres are in contact with each other, wherein the electrically conductive textile fabric is provided with electrical contacts, for producing electrically heatable patches according to one of Claims 1 to 7.

11. An electrically heatable patch according to one of Claims 1 to 7 for use in local heat therapy, for increasing skin permeation for a pharmaceutical active ingredient, preferably for a pharmaceutical active ingredient contained in the patch, and/or in the transdermal administration of an active ingredient, preferably a pharmaceutical active ingredient contained in the patch.

## Revendications

1. Pansement électriquement chauffable comprenant une couche de contact avec la peau autocollante et une structure plate textile électriquement conductrice, la structure plate textile électriquement conductrice étant dotée de contacts électriques, et la structure plate textile électriquement conductrice étant un tissu, un aiguilleté, un tricot, une natte, une nappe bidirectionnelle ou multidirectionnelle, un feutre ou un voile en fibres, dans lequel des fibres électriquement conductrices sont en contact les unes avec les autres, **caractérisé en ce que** la structure plate textile électriquement conductrice forme la couche arrière du pansement et le pansement électriquement chauffable contenant au moins une substance active pharmaceutique.

2. Pansement selon la revendication 1, **caractérisé en ce que** la structure plate textile est choisie dans le groupe des structures plates textiles, qui est composé
a) de fibres exclusivement électriquement conductrices, de préférence de fibres métalliques ;
b) de mélanges de fibres électriquement conductrices et de fibres électriquement non conductrices ; et/ou
c) d'une ou plusieurs structures plates textiles électriquement non conductrices, qui sont apprêtées avec un ou des liants collants, électriquement conducteurs, et des particules électriquement conductrices, de préférence des particules métalliques, qui sont fixées en trois dimensions et ultérieurement dans la structure plate textile.

3. Pansement selon la revendication 1 ou 2, **caractérisé en ce que** la structure plate électriquement conductrice se présente incorporée dans une couche de résine ou une couche de polymère.

4. Pansement selon la revendication 3, **caractérisé en ce que** la couche de résine ou la couche de polymère présente au moins une charge électriquement conductrice.

5. Pansement selon l'une des revendications précédentes, **caractérisé en ce qu'**il présente une source de tension, de préférence une source de courant continu.

6. Pansement selon l'une des revendications précédentes, **caractérisé en ce qu'**il présente un dispositif électronique de commande et de réglage permettant de maintenir la température de la structure plate textile constante.

7. Pansement selon l'une des revendications précédentes, caractérisé en ce la substance active pharmaceutique est choisie dans le groupe qui comprend des anti-inflammatoires non stéroïdiens (AINS), des anti-cholinergiques, des parasympatholytiques, des anti-mycosiques, des inhibiteurs de la MAO-B, des antagonistes de sérotonine, des agonistes du récepteur alpha2, des photosensibilisants, des hormones et/ou des protéines.

8. Procédé de production d'un pansement électriquement chauffable selon l'une des revendications 1 à 7, comprenant les étapes suivantes :
- mettre à disposition une structure plate textile électriquement conductrice, la structure plate textile électriquement conductrice étant un tissu, un aiguilleté, un tricot, une natte, une nappe bidirectionnelle ou multidirectionnelle, un feutre ou un voile en fibres, dans lequel les fibres électriquement conductrices sont en contact les unes avec les autres,
- stratifier la structure plate textile électriquement conductrice sur une couche de contact avec la peau autocollante,
- individualiser les pansements individuels, et
- apposer les contacts sur la face non stratifiée de la structure plate textile électriquement conductrice.

9. Procédé selon la revendication 8, comprenant en outre l'étape consistant à doter le stratifié d'au moins une autre couche.

10. Utilisation d'une structure plate textile électriquement conductrice, la structure plate textile électriquement conductrice étant un tissu, un aiguilleté, un tricot, une natte, une nappe bidirectionnelle ou multidirectionnelle, un feutre ou un voile en fibres, dans lequel des fibres électriquement conductrices sont en contact les unes avec les autres, la structure plate textile électriquement conductrice étant dotée de contacts électriques, pour la production de pansements électriquement chauffables selon l'une des revendications 1 à 7.

11. Pansement électriquement chauffable selon l'une des revendications 1 à 7 pour utilisation dans la thérapie thermique locale, pour l'augmentation de la perméation de la peau pour une substance active pharmaceutique, de préférence pour une substance active pharmaceutique contenue dans le pansement, et/ou l'administration transdermique d'une substance active, de préférence d'une substance active pharmaceutique contenue dans le pansement.
